# EUROPEAN PATENT APPLICATION

(11) **EP 3 533 778 A1**
(43) Date of publication of application: **04.09.2019**
(21) Application number: 17863714.6
(22) Date of filing: 13.07.2017
(51) Int. Cl.: C07C 67/303, C07C 69/75, C07B 61/00, B01J 23/46

(54) **PRODUCTION METHOD FOR BIS(2-HYDROXYETHYL) 1,4-CYCLOHEXANEDICARBOXYLATE**

(30) Priority: 28.10.2016 JP 2016211755
(71) Applicant: Jeplan, Inc., Tokyo 1006025 (JP)
(72) Inventor: ITO, Hiroshi, Kizugawa-shi Kyoto 619-0225 (JP); IMANAKA, Hiroshi, Funabashi-shi Chiba 273-0853 (JP); INADA, Shuji, Suita-shi Osaka 565-0873 (JP)
(74) Representative: Hoffmann Eitle
(86) International application number: PCT/JP2017/025539
(87) International publication number: WO 2018/078960

(57) **Abstract**

A process for producing 1,4-cyclohexanedicarboxylic acid bis (2-hydroxyethyl) (BHEC) that make it possible to obtain (BHEC) simply and with high yield. The process comprises a step of hydrogenating the benzene ring of terephthalic acid bis (2-hydroxyethyl) (BHET) by reacting BHET with hydrogen gas in the presence of a ruthenium catalyst. It is preferable that the hydrogenation of the benzene ring of BHET is carried out using hydrogen gas having a partial pressure of 1-10 MPa at temperatures of 80-200 °C.

## Description

### BACKGROUND OF THE INVENTION

### Technical Field

The present invention relates to a process for producing 1,4-cyclohexanedicarboxylic acid bis (2-hydroxyethyl).

### Background Art

Consumption of polyethylene terephthalate (hereinafter referred to as "PET") products is increasing year by year, and improving the recycling rate of waste PET products is an essential proposition on a global scale. As a recycling method of waste PET products, it is known, for example, a method of obtaining terephthalic acid bis (2-hydroxyethyl) (hereinafter referred to as "BHET") from a waste PET product and recycling it as a raw material of PET.

BHET is also used as a raw material for 1,4-cyclohexanedicarboxylic acid bis (2-hydroxyethyl) (hereinafter referred to as "BHEC") besides recycling as a raw material for PET. BHEC can be produced, for example, by reacting BHET with hydrogen (hydrogen gas) to hydrogenate (nuclear hydrogenate) the benzene ring of BHET. When BHEC is used as a raw material of a polyester resin, the heat resistance, transparency, weather resistance and moldability of the polyester resin to be produced can be improved. BHEC can also be used as a chain extender (diol component) of polyurethane resin, or as a raw material for acrylic resin or epoxy resin. As a method for obtaining BHEC from BHET, there is, for example, a method disclosed in Patent Document 1.

According to the method disclosed in Patent Document 1, BHEC is produced by reacting BHET with hydrogen gas in the presence of a palladium-carried catalyst to perform nuclear hydrogenation of BHET. However, in the above method, a large amount of by-products other than BHEC was produced, making it difficult to increase the yield of BHEC.

### PRIOR ART DOCUMENTS

### Patent Documents

Patent Document 1: CN104003840 (A)

### SUMMARY OF THE INVENTION

### Problem to be solved by The Invention

The present invention has been made in view of the above problems, and aims to provide a process for simply producing 1,4-cyclohexanedicarboxylic acid bis (2-hydroxyethyl) (BHEC) that make it possible to obtain BHEC in high yield.

### Means to solve The Problem

The present invention includes:
(1) A process for producing 1,4-cyclohexanedicarboxylic acid bis (2-hydroxyethyl) comprising a step of hydrogenating the benzene ring of terephthalic acid bis (2-hydroxyethyl) by reacting the terephthalic acid bis (2-hydroxyethyl) with hydrogen gas in the presence of a ruthenium catalyst.
(2) The process according to the above (1), wherein the hydrogenation is carried out using a reaction gas having a partial pressure of hydrogen gas of 1 to 10 MPa.
(3) The process according to the above (1) or (2), wherein the hydrogenation is carried out at temperatures of 80 to 200 °C.
(4) The process according to any one of the above (1) to (3), wherein the weight ratio between the terephthalic acid bis (2-hydroxyethyl) and the ruthenium catalyst is 100:0.1 to 100:10.
(5) The process according to any one of the above (1) to (4), wherein the hydrogenation is carried out in a state that the terephthalic acid bis (2-hydroxyethyl) and the ruthenium catalyst are mixed in a liquid medium.
(6) The process according to the above (5), wherein the liquid medium contains at least one of a diol and an aprotic polar solvent.
(7) The method according to the above (6), wherein the liquid medium contains at least one of ethylene glycol, propylene glycol, butanediol, N, N-dimethylformamide, dimethylsulfoxide and 1,3-dimethyl-2-imidazolidinone.
(8) The process according to any one of the above (1) to (7), wherein by-products other than the 1,4-cyclohexanedicarboxylic acid bis (2-hydroxyethyl) are produced at less than 10 wt% during the hydrogenation.
(9) The process according to any one of the above (1) to (8), wherein the ruthenium catalyst comprises a carrier containing at least one of carbon, alumina, silica, titania, magnesia and zirconia, and a ruthenium carried on the carrier.
(10) The process according to any one of the above (1) to (9), wherein the terephthalic acid bis (2-hydroxyethyl) is obtained by depolymerizing a raw material containing polyethylene terephthalate.

### Effect of The Invention

According to the present invention, 1,4-cyclohexanedicarboxylic acid bis (2-hydroxyethyl) (BHEC) can be easily obtained in high yield from terephthalic acid bis (BHET). In addition, the recycling rate of waste PET products can be improved by using BHET obtained from a waste polyethylene terephthalate (PET) product as a raw material.

### BEST MODE FOR CARRYING OUT THE INVENTION

Hereinafter, preferred embodiments of the process for producing 1,4-cyclohexanedicarboxylic acid bis (2-hydroxyethyl) according to the present invention will be described in detail.

The process for producing 1,4-cyclohexanedicarboxylic acid bis (2-hydroxyethyl) (BHEC) of the present invention comprises a step of hydrogenating (nuclear hydrogenating) the benzene ring of terephthalic acid bis (2-hydroxyethyl) (BHET) by reacting BHET with hydrogen gas in the presence of a ruthenium catalyst.

In the specification, unless otherwise stated, the "ruthenium catalyst" means a ruthenium metal simple substance, a ruthenium oxide or a ruthenium alloy, or a ruthenium carried carrier catalyst on which is carried such a metal, oxide or alloy.

More specifically, the BHEC producing process of the embodiments according to the present invention includes steps of preparing a raw material containing BHET (raw material preparation step) and nuclear hydrogenating BHET by putting the raw material and the ruthenium catalyst into the autoclave and reacting BHET with hydrogen gas in the presence of the ruthenium catalyst to obtain BHEC (nuclear hydrogenation step). Each step will be described in turn below.

### <Raw Material Preparation Step>

First, a raw material containing BHET is prepared.

As a raw material, BHET containing products available in various ways can be used, such as commercially available polyethylene terephthalate (PET) products, waste PET products, commercially available BHET or synthesized BHET. Among them, from the viewpoint of improving the recycling rate of waste PET products, it is preferable to use waste PET products. Examples of waste PET products include waste PET bottles, waste PET films, waste PET fibers, waste PET industrial materials, etc.

As a method of obtaining BHET from a waste PET product, for example, a method of depolymerizing PET in ethylene glycol (hereinafter referred to as "EG") is known (see, for example, JP2000-53802A and JP2008-88096A). According to such a method, it is possible to obtain high purity BHET from an inexpensive waste PET product in high yield. In addition, since such a method is chemical recycling that effectively utilizes resources, it is also a technology that greatly contributes to global environment protection.

A method of synthesizing BHET includes a method of adding ethylene oxide to terephthalic acid, a method of transesterification reaction of dimethyl terephthalate (DMT) and ethylene glycol, and the like.

The content of BHET (purity of BHET) in the raw material is preferably sufficiently high from the viewpoint of improving the yield of BHEC to be produced. Specifically, the content of BHET in the raw material is preferably 90 wt% or more, more preferably 92 wt% or more, still more preferably 95 wt% or more. By using a raw material having the purity of BHET in the above ranges, BHEC of higher purity can be obtained in good yield.

### <Nuclear Hydrogenation Step>

The thus prepared raw material is charged (supplied) with the ruthenium catalyst into a vessel (reactor). Thereafter, a reaction gas containing hydrogen gas is filled in the reactor, to react BHET with hydrogen gas in the presence of the ruthenium catalyst. As a result, the benzene ring of BHET is hydrogenated (also called "nuclear hydrogenated") to obtain BHEC.

As the reactor, an autoclave equipped with a stirrer, for example, can be used.

The method disclosed in Patent Document 1 as referred to above is known as a process for producing BHEC by nuclear hydrogenation of BHET. In the method disclosed in Patent Document 1, however, the reaction between BHET and hydrogen gas occurs in the presence of a palladium-carried catalyst to nuclear hydrogenate BHET. Therefore, not only BHEC in which the benzene ring is hydrogenated, but also 1,4-cyclohexanedimethanol (hereinafter referred to as "CHDM") in which both of the benzene ring and the ester group (-COOCH₂CH₂OH)) are generated. Thus, in the method of Patent Document 1, it is difficult to control the formation of CHDM necessarily generated by hydrogenation of an ester group. Therefore, a large amount of by-products (e.g., CHDM) other than BHEC are produced, making it difficult to sufficiently increase the yield of BHEC.

The present inventors examined and analyzed the conventional process and sought to selectively hydrogenate the benzene ring of BHET to obtain high purity BHEC in good yield. As a result, it was found that by reacting BHET and hydrogen gas in the presence of a ruthenium catalyst, the benzene ring of BHET is selectively hydrogenated, and hydrogenation of the ester group can be suppressed. That is, it was found that, in the presence of ruthenium, a high purity BHEC can be obtained in good yield by the reaction of BHET and hydrogen gas, and the present invention has been completed accordingly.

The ruthenium catalyst acts as a catalyst for efficiently hydrogenating the benzene ring of BHET. In the BHEC production method of the present invention, the ruthenium metal can be used as it is, but it is preferably used in the form of a ruthenium-supported carrier catalyst on which ruthenium is supported. The carrier adsorbs ruthenium and disperses the points acting as a catalyst (catalyst active points) on the surface of the carrier. As a result, the contact area between the catalyst active points and BHET can be increased, so that the efficiency of nuclear hydrogenation of BHET can be further enhanced. Moreover, by supporting ruthenium on a carrier, durability of ruthenium as a catalyst can be enhanced.

Ruthenium catalyst can carry not only ruthenium alone but also a metal other than ruthenium in the form of a ruthenium alloy. Such a metal includes platinum group metal of Rh, Pd, Os, Ir or Pt, metal element such as Ni, Co, Fe, Zn, Cu, Mn, Pd, Cd, Cr, Ag, Au, Hg, Ga, In, Ge, Sn, A Ti, Al, Si or the like, an alkaline earth metal element of Ca, Mg, Gr or Ba, and an alkaline metal of Li, Na, K, Rb or Cs.

Further, as a carrier which carries ruthenium, it is preferable to contain at least one of carbon (activated carbon), alumina, silica, titania, magnesia, zirconia, silica alumina, zeolite, clay, kaolin, talc and bentonite. Among them, a carrier containing at least one of carbon, alumina, silica, titania, magnesia and zirconia is more preferable, and a carrier containing carbon being particularly preferable.

The amount (content) of ruthenium supported on the carrier is not particularly limited, but preferably about 0.1-10 wt%, and more preferably about 0.5-10 wt%. When the amount of ruthenium carried on the carrier is within the above range, it is possible to obtain a catalyst in which the catalyst active sites are uniformly dispersed over the surface of the carrier while minimizing the use amount of expensive ruthenium. By using such a ruthenium carried carrier catalyst, BHET can be nuclear hydrogenated efficiently at low cost, and high purity BHEC can be obtained in good yield. In addition, since such a ruthenium-carried carrier catalyst has high durability as described above, it can be recycled as a catalyst by recovering the carrier separately.

From the viewpoint of improving the catalytic action of the ruthenium catalyst, an activation treatment of the ruthenium catalyst may be carried out in which the ruthenium catalyst is previously heated in flows of gas such as hydrogen gas, nitrogen gas, argon gas, carbon dioxide gas and the like. The treatment temperature in that occasion is preferably about 50 to 700 °C, and more preferably about 80 to 600 °C. When using a ruthenium-supported carrier catalyst, for example, by heating the catalyst at a temperature within the above temperature range, the catalyst active points of ruthenium is more uniformly dispersed over the surface of the carrier, so that the catalytic characteristics of the ruthenium-carried carrier can be further enhanced. The time of the activation treatment can be appropriately adjusted depending on the treatment temperature and the amount of ruthenium supported on the carrier, but it can be set to, for example, about 0.1 to 100 hours.

The charging ratio between BHET and ruthenium catalyst charged into the autoclave (reactor) is not particularly limited, but it is preferably 100: 0.1 to 100: 10 in terms of weight ratio of BHET and catalyst, and more preferably 100: 1 to 100: 5. This makes it possible to nuclear hydrogenate BHET efficiently in a shorter time and reliably suppress side reactions (such as hydrogenation reaction of ester group of BHET) other than nuclear hydrogenation reaction of BHET. As a result, BHEC with higher purity can be obtained in good yield.

Incidentally, in the nuclear hydrogenation reaction of BHET, it is desirable to increase the contact between BHET and hydrogen and/or ruthenium catalyst as much as possible. However, since the melting point of BHET is relatively high (about 110 °C), a large amount of heat is required to increase the fluidity of BHET by heating BHET and hydrogen and/or ruthenium catalyst in the reactor. In this context, in order to improve the fluidity of BHET, it is effective to dissolve or disperse BHET in a liquid medium. By using the liquid medium, the heat of reaction is lowered, so that side reactions can be suppressed more reliably. When using the liquid medium in this step, it is preferable to charge the raw material, the ruthenium catalyst and the liquid medium into the autoclave and stir by an agitator of the autoclave so that the nuclear hydrogenation of BHET can be carried out in the state that the raw material and the ruthenium catalyst are dissolve or disperse in the liquid medium.

The liquid medium usable in this nuclear hydrogenation step is not particularly limited as long as it does not adversely affect the nuclear hydrogenation reaction of BHET, but it is preferable that it contains at least one kind of diol and aprotic polar solvent. Since these liquid media have high solubility in BHET, the fluidity of BHET can be improved. In addition, it is possible to suppress the occurrence of side reactions such as polymerization during the nuclear hydrogenation reaction of BHET. Therefore, by using such a liquid medium, BHEC with higher purity can be obtained in good yield.

The diol is not particularly limited, but it is preferable to include at least one kind of ethylene glycol, propylene glycol, butane diol and the like. Also, the aprotic polar solvent is not particularly limited, but it is preferable to include at least one of N, N-dimethyl formamide, dimethyl sulfoxide and 1,3-dimethyl-2-imidazolidinone. Among these liquid mediums, ethylene glycol is particularly preferable from the viewpoint that it is a diol constituting BHET, the solubility of BHET being high and the effect of suppressing the occurrence of side reaction such as polymerization being high.

Although the amount (amount of use) of the liquid medium to be charged into the autoclave is not particularly limited, it is preferably 5:95 to 90:10, and more preferably 10:90 to 70:30 in terms of the weight ratio between BHET and the liquid medium. When the amount of the liquid medium used falls within the above range, the nuclear hydrogenation reaction of BHET proceeds efficiently and side reactions can be effectively suppressed. As a result, the productivity of BHEC can be improved with higher yield of BHEC.

After introducing the raw material and the ruthenium catalyst into an autoclave equipped with a stirrer, the interior of the autoclave is evacuated (vacuum degassed) and then filled with a reaction gas containing hydrogen gas, or an air in the autoclave is substituted by reaction gas containing hydrogen gas to thereby react BHET with hydrogen gas in the presence of ruthenium.

Note that the gas substitution operation and vacuum degassing operation may be performed in combination, or these operations may be repeated alternately and repeatedly. The reaction gas to be filled in the autoclave may be a single hydrogen gas, or a mixed gas of nitrogen, argon or carbon dioxide gas and hydrogen gas.

When using a mixed gas, the partial pressure of hydrogen gas (the total pressure when using hydrogen gas alone) is not particularly limited, but preferably about 1-10 MPa, more preferably about 2 to 8 MPa, and further more preferably about 3 to 6 MPa. When the partial pressure of hydrogen gas is within the above range, hydrogen gas more selectively reacts with the benzene ring of BHET, and nuclear hydrogenation of BHET proceeds more rapidly. As a result, BHEC can be obtained efficiently. Furthermore, with such a relatively low partial pressure, since the burden on the autoclave is reduced, it is possible to manufacture BHEC repeatedly in the same facility for a long period of time, so that the running cost can be suppressed. Note that, during the nuclear hydrogenation of BHET, it is preferable to supply hydrogen gas continuously or intermittently into the autoclave so that the partial pressure of hydrogen gas in the autoclave can become constant.

The reaction temperature (temperature in the autoclave) at the time when BHET is reacted with hydrogen gas is preferably about 80-200 °C, more preferably about 85-180 °C, and further more preferably about 80-170 °C. If the reaction temperature is within the above range, hydrogen gas more selectively reacts with the benzene ring of BHET with the nuclear hydrogenation of BHET proceeding promptly. In addition, when the reaction temperature is within the above range, it is possible to more reliably suppress side reactions, such as hydrogenation reaction of ester group of BHET, etc., other than the nuclear hydrogenation reaction of BHET. As a result, high purity BHEC can be obtained in higher yield. Also, with such relatively low reaction temperature, since the burden on the autoclave is reduced, the running cost can be suppressed.

The reaction time of BHET and hydrogen gas is appropriately adjusted depending on the type of the ruthenium catalyst to be used, the charging ratio of the raw material and ruthenium, the pressure of the reaction gas (partial pressure of hydrogen gas), the reaction temperature, etc., and is thus not particularly limited, but may be about 2 to 20 hours, for example.

The reaction for obtaining BHEC can be carried out by the use of a batch type reactor, a continuous type reactor, or a reactor which is a combination of a batch type and a continuous type. As the batch type hydrogenation apparatus, a reactor (autoclave) equipped with a stirrer and a cooler such as a total reflux condenser can be used. Also, as the continuous type hydrogenation apparatus, a known continuous fixed bed reactor can be used.

After carrying out the hydrogenation reaction as described above, the autoclave is cooled down to room temperature (about 20 °C) as needed. Thereafter, the crude BHEC can be obtained by separating the ruthenium catalyst from the reaction system in the autoclave. The content of BHEC in such crude BHEC is preferably 90 wt% or more. According to the BHEC production process of the present invention, formation of by-products is suppressed, and BHEC of high purity can be obtained.

The obtained crude BHEC can be purified by a known separation and purification process such as distillation or recrystallization.

The ruthenium catalyst separated from the above reaction system can be reused repeatedly. That is, a new raw material and the ruthenium catalyst separated and recovered from the above reaction system are charged together into the autoclave, and a nuclear hydrogenation of BHET is carried out again and again in the same manner as those stated above to obtain BHEC. As described above, even when the used ruthenium catalyst is recycled, BHET can be nuclear hydrogenated to obtain high purity BHEC with high yield.

Although the process for producing 1,4-cyclohexanedicarboxylic acid bis (2-hydroxyethyl) of the present invention has been described above, the present invention is not limited thereto and one or more steps for any desired purposes may be added to the process.

### EXAMPLES

Hereinafter, specific examples of the present invention will be described.

The raw material containing terephthalic acid bis (2-hydroxyethyl) (BHET) used in each of examples and comparative examples shown below was obtained from a polyethylene terephthalate (PET) bottle by the following process (de-polymerization) :

### 1. Preparation of raw materials containing BHET

First, a used PET bottle including 10 wt% of a colored PET bottle was charged into a wet pulverizer provided with cutter blades and pulverized into flakes having an average size of 8 mm square. Next, the flakes were taken out, and then 200 kg of a 4 wt% NaOH aqueous solution was added to 50 kg of the flakes, heated to 80-85 °C, and stirred to wash the flakes for 30 minutes. Thereafter, the flakes were separated into solid and liquid and washed with water to remove the NaOH component. Next, the flakes were rinsed with pure water, centrifuged and dehydrated, and then vacuum dried.

30 kg of the dried flakes, 168 kg of ethylene glycol (hereinafter referred to as "EG") and 108 g of Mg(OH)₂ as a catalyst were supplied to an autoclave equipped with a stirrer and a rectification column to make a mixed solution. After nitrogen substitution and vacuum degassing of the autoclave were repeatedly performed three times, a de-polymerization reaction was carried out at 197-200 °C for 1.5 hours under normal pressure while a fraction having a boiling point lower than that of EG was removed outside the system from the top of the rectification column. After completion of the de-polymerization reaction, the temperature of the mixed solution was cooled to 97-98 °C in 45 minutes while stirring. After cooling, hot coarse particles were filtered out with a stainless steel wire mesh filter having an average pore diameter of 60 µm and suspended matters and precipitates in the mixed solution were filtered out with a 1 µm fiber felt bag filter.

Next, the mixed solution was supplied to a jacket type vertical thin film evaporator equipped with a stirrer (Model "RF-6" of UIC GmbH) to evaporate the low boiling point component of the mixed solution and obtain a first stage concentrate under conditions of a jacket temperature being 150 °C and an internal pressure of the evaporator main body being 533 Pa. The first stage concentrated solution was again supplied to the jacket type vertical thin film evaporator equipped with a stirrer (Model "RF-6" of UIC GmbH), to evaporate the remaining low boiling point component in the first stage concentrated solution and obtain a second stage concentrate (high boiling point component) under conditions of the jacket temperature being 150 °C and the internal pressure of the evaporator main body being 133 Pa.

The second stage concentrate (high-boiling point component) was then supplied to a jacket type vertical thin film evaporator equipped with a stirrer (Model "RF-6" of UIC GmbH) and the weight ratio of the BHET evaporated fraction to evaporation residue (still residue) was set to be 8:2 under the setting conditions that a jacket temperature was 202 °C and an internal pressure of the evaporator main body was 13 Pa.

27 kg of the crude BHET obtained was dissolved in 108 kg of hot water of 70 °C. This aqueous solution was supplied to a crystallization tank, rapidly cooled from 70 °C to 65 °C, slowly cooled from 65 °C to 40 °C for 5 hours, and gradually cooled from 40 °C to 20 °C for 2 hours to thereby crystallize and precipitate BHET crystals. Thereafter, a solid-liquid separation of the aqueous solution was performed using a vertical type centrifugal separator while maintaining the aqueous solution at 20 °C. The purified BHET remained in the centrifugal separator was washed with pure water of 15 °C and then subjected to solid-liquid separation again.

The purified BHET separated as described above was analyzed by a gas chromatography with the result that the purity was 96.3 wt%, the acid value of purified BHET was 2.3 mg KOH/g and the melting point was 111 °C. In this step and in each of the following steps, columns and measurement temperature conditions used for the gas chromatography analysis are as follows:
- Column: DB-1 (made by Agilent Technologies, 30 m x 0.25 mm, film thickness:0.25 µm)
- Temperature conditions: raising from 150 °C to 300 °C at 5 °C/min and held at 300 °C for 15 minutes
- Carrier gas: helium

### 2. Nuclear hydrogenation of BHET

### (Example 1)

80 g of the purified BHET (raw material containing BHET) obtained above, 2.4 g of a carbon carrier catalyst (5% Ru/C) supporting 5 wt% ruthenium and 120 g of EG as a solvent (liquid medium) were charged into a 0.5 L electromagnetic stirring type autoclave. The weight ratio of the ruthenium-carried carbon carrier to BHET was 3.1 wt%, with the weight ratio of EG to BHET being 155.8 wt%.

After vacuum degassing the inside of the autoclave, a nuclear hydrogenation of BHET was carried out with the temperature in the autoclave being 130 °C while hydrogen gas was continuously supplied into the autoclave so that the partial pressure of hydrogen gas in the autoclave could be maintained at 3 MPa.

In the nuclear hydrogenation of this example, hydrogen absorption by BHET ceased after 2.1 hours from the time when the temperature in the autoclave reached 130 °C.

Thereafter, the interior of the autoclave was cooled to room temperature, and the carbon carrier catalyst carrying ruthenium was filtered out (separated) from the reaction mixture to obtain a mixed solution of the nuclear hydrogenation reaction. As a result of gas chromatography analysis of the reaction mixed solution, the purity of BHEC excluding EG was 95.3 wt%. Table 1 shows the nuclear hydrogenation conditions and the composition of the reaction mixed solution of this example and each of the following examples and comparative examples as described below.

### (Example 2)

4.5 g (weight of the catalyst containing the reaction mixed solution) of the ruthenium carried carbon carrier catalyst which was recovered from the reaction mixed solution in Example 1 was, after filtered out from the reaction mixed solution, reused and again subjected to nuclear hydrogenation in the same manner as in Example 1. In the nuclear hydrogenation of this example, hydrogen absorption of BHET ceased after 3.4 hours from the time when the temperature in the autoclave reached 130 °C. Thereafter, the reaction mixed solution obtained in the same post-treatment as in Example 1 was analyzed by gas chromatography. As a result, the purity of BHEC excluding EG was 96.2 wt%.

### (Example 3)

Nuclear hydrogenation was carried out in the same manner as in Example 1 except that the composition and the nuclear hydrogenation condition of the reaction system shown in Table 1 were changed. In the nuclear hydrogenation of this example, hydrogen absorption by BHET ceased after 1.2 hours from the time when the temperature in the autoclave reached 150 °C. Thereafter, the reaction mixed solution obtained in the same post-treatment as in Example 1 was analyzed by gas chromatography. As a result, the purity of BHEC excluding EG was 91.9 wt%.

### (Comparative Example 1)

Nuclear hydrogenation was carried out in the same manner as in Example 1 except for changing the composition and the nuclear hydrogenation conditions of the reaction system as shown in Table 1. In this comparative example, hydrogen absorption of BHET ceased after 4 hours after the time when the temperature in the autoclave reached 150 °C. Thereafter, the reaction mixed solution obtained in the same post-treatment as in Example 1 was analyzed by gas chromatography. As a result, the purity of BHEC excluding EG was 83.1 wt%.

### (Comparative Example 2)

Nuclear hydrogenation was carried out in the same manner as in Example 1 except that the composition and the nuclear hydrogenation condition of the reaction system were changed as shown in Table 1. However, in this Comparative Example, the hydrogen absorption of BHET was slow, so that the nuclear hydrogenation of BHET did not sufficiently proceed. For this reason, the reaction was interrupted after 8 hours elapsed from the time when the temperature in the autoclave reached 200 °C.

After interrupting the reaction, the reaction mixed solution obtained in the same post-treatment as in Example 1 was subjected to gas chromatography analysis. The purity of BHEC excluding EG was 55.0 wt%.

The composition (EG excluded from calculation) of the reaction mixed solution obtained in each of the examples and comparative examples, after the gas chromatography analysis, is shown in Table 1. What the shortened terms in Table 1 represent are as follows:
5%Ru/C: Carbon carrier catalyst carrying 5 wt% of ruthenium
5%Pd/C: Carbon carrier catalyst carrying 5 wt% of palladium
Stabilized Ni: Stabilized Ni catalyst

**[Table 1]**

| | Example1 | Example2 | Example3 | Comparative Example1 | Comparative Example2 |
|---|---|---|---|---|---|
| BHEC content in raw material (g) | 77 | 77 | 77 | 77 | 77 |
| Catalyst type | 5%Ru/C | Recovered Catalyst | 5%Ru/C | 5%Pd/C | Stabilized Ni |
| Amount of catalyst use (wt% to BHET) | 3.1 | (Reuse) | 2.1 | 10.4 | 10.4 |
| Amount of solvent (EG) use (wt% to BHET) | 155.8 | 155.8 | 103.8 | 155.8 | 103.8 |
| Reaction temperature (°C) | 130 | 130 | 150 | 150 | 150 |
| Hydrogen partial pressure (MPa) | 3 | 3 | 4 | 3 | 8 |
| Hydrogen absorption time (h) | 2.1 | 3.4 | 1.2 | 4 | 8 |

| COMPOSITION OF CRUDE BHEC (GC%) | | | | | |
|---|---|---|---|---|---|
| Low-boiling substances | 0.5 | 0.4 | 2.7 | 6 | 31.8 |
| CHDM | 4 | 2.9 | 5.1 | 10.5 | 2.1 |
| BHEC | 95.3 | 96.2 | 91.9 | 83.1 | 55.0 |
| BHET | 0 | 0.2 | 0.1 | 0 | 10.6 |
| High-boiling substances | 0.2 | 0.2 | 0.2 | 0.4 | 0.5 |

As shown in Table 1, the BHEC purity of the reaction mixed solution obtained in Examples 1 to 3 was 90 wt% or more, and thus BHEC with high purity could be obtained from the raw material containing BHET. In contrast, the BHEC purity of the reaction mixed solution obtained in Comparative Examples 1 and 2 was low. In addition, the crude BHEC obtained in Comparative Example 1 contained a large amount of CHDM as a by-product. Further, the crude BHEC obtained in Comparative Example 2 contained a large amount of unreacted BHET and low-boiling substances. In order to obtain high purity BHEC from the reaction mixed solution of Comparative Example 1 and Comparative Example 2, a large burden will be placed on the purification process and the resultant yield would be lowered. Therefore, it can be said that the BHEC production processes of Examples 1 to 3 have higher BHEC purity of the obtained reaction mixed solution than Comparative Examples 1 and 2, and is thus industrially advantageous.

## Claims

1. A process for producing 1,4-cyclohexanedicarboxylic acid bis (2-hydroxyethyl) comprising a step of hydrogenating the benzene ring of terephthalic acid bis (2-hydroxyethyl) by reacting the terephthalic acid bis (2-hydroxyethyl) with hydrogen gas in the presence of a ruthenium catalyst.

2. The process according to claim 1, wherein the hydrogenation is carried out using a reaction gas having a partial pressure of hydrogen gas of 1-10 MPa.

3. The process according to claim 1 or 2, wherein the hydrogenation is carried out at temperatures of 80-200 °C.

4. The process according to any one of claims 1 to 3, wherein the weight ratio of the terephthalic acid bis (2-hydroxyethyl) to the ruthenium catalyst is 100 : 0.1 - 100 : 10.

5. The process according to any one of claims 1 to 4, wherein the hydrogenation is carried out in a state that the terephthalic acid bis (2-hydroxyethyl) and the ruthenium catalyst are mixed in a liquid medium.

6. The process according to claim 5, wherein the liquid medium contains at least one of a diol and an aprotic polar solvent.

7. The method according to claim 6, wherein the liquid medium contains at least one of ethylene glycol, propylene glycol, butanediol, N, N-dimethylformamide, dimethylsulfoxide and 1,3-dimethyl-2-imidazolidinone.

8. The process according to any one of claims 1 to 7, wherein by-products other than the 1,4-cyclohexanedicarboxylic acid bis (2-hydroxyethyl) are produced at less than 10 wt% during the hydrogenation.

9. The process according to any one of claims 1 to 8, wherein the ruthenium catalyst comprises a carrier containing at least one of carbon, alumina, silica, titania, magnesia and zirconia, and a ruthenium carried on the carrier.

10. The process according to any one of claims 1 to 9, wherein the terephthalic acid bis (2-hydroxyethyl) is obtained by depolymerizing a raw material containing polyethylene terephthalate.
